(19) Europäisches Patentamt
European Patent Office
Office européen des brevets

(11) Veröffentlichungsnummer: **0 245 772 B1**

(12) # EUROPÄISCHE PATENTSCHRIFT

(45) Veröffentlichungstag der Patentschrift: **08.11.89**

(51) Int. Cl.⁴: **C07C 9/22**, A01N 27/00

(21) Anmeldenummer: **87106596.7**

(22) Anmeldetag: **07.05.87**

(54) **5,9-Dimethylheptadecan Verfahren und Mittel zur Bekämpfung der Fleckenminiermotte Leucoptera scitella.**

(30) Priorität: **10.05.86 DE 3615854**

(43) Veröffentlichungstag der Anmeldung:
**19.11.87 Patentblatt 87/47**

(45) Bekanntmachung des Hinweises auf die Patenterteilung:
**08.11.89 Patentblatt 89/45**

(84) Benannte Vertragsstaaten:
**AT CH DE ES FR GR IT LI**

(56) Entgegenhaltungen:
**NATURWISSENSCHAFTEN, Band 74, Nr. 3, 1987, Seiten 143-144, Springer-Verlag, Berlin, DE; W. FRANCKE et al.: "Identification of 5,9-dimethylheptadecane as a sex pheromone of the moth Leucoptera scitella"**

(73) Patentinhaber: **BASF Aktiengesellschaft, Carl-Bosch-Strasse 38, D-6700 Ludwigshafen(DE)**

(72) Erfinder: **Francke, Wittko, Prof. Dr., Am Vorwerksbusch, D-2057 Reinbek(DE)**

**EP 0 245 772 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft die Verbindung 5,9-Dimethylheptadecan und dessen Verwendung zur Bekämpfung der Fleckenminiermotte.

Die Fleckenminiermotte Leucoptera (= Cemiostoma) scitella (Zell.) aus der Familie der Lyonetiidae verursacht an verschiedenen Obstbäumen Schäden. Andere deutschsprachige Bezeichnungen sind z.B. auch Münzen- oder Pfennigminiermotte.

Dieser Kleinschmetterling tritt in europäischen Obstbaugebieten sporadisch auf, aber besonders in Frankreich, Italien und Ungarn wird von erheblichen Schäden insbesondere in Apfelkulturen berichtet, vgl.

HEINZE, 1978: Schädlinge und Krankheiten im Obst- und Weinbau, S. 165;
CELLI 1975, 1984: Instituto di Entomologia, Universita di Bologna, Italy;
CHAMBON 1981: Phytoma 331, 18–20 (Frankreich);
DULIC, INJAC, 1981: Zastita Bilja 32, 409–426 (Jugoslawien);
BALAZS 1983: Novenyvedelem 19, 305–306 (Ungarn).

Die wirtschaftliche Bedeutung des Schädlings besteht darin, daß mehr oder weniger große Teile der Blattoberfläche zerstört werden und für die Assimilation ausfallen. Bei starkem Befall können die Blätter vorzeitig abgestoßen werden, Notreife der Früchte kann eine Folge sein. Bei Blattfall wird die Einlagerung ausreichender Reservestoffe für den Winter verhindert.

Die Bekämpfung der Miniermotten ist aufgrund ihrer versteckten Lebensweise nicht einfach. Eingesetzt werden können insektizide Wirkstoffe aus der Gruppe der Phosphorsäureester mit ausgeprägter Tiefenwirkung. Sämtliche derartige Produkte haben jedoch den Nachteil, daß sie keine selektive Wirkung zeigen und in vielen Fällen auch warmblütertoxisch sind.

Eine zuverlässige Methode zur Erkennung des Auftretens dieses schädlichen Schmetterlings oder eine selektive Bekämpfungsmethode waren aber bisher nicht bekannt und somit eine gezielte Bekämpfung nicht möglich.

Es ist an sich bekannt, daß bei Schmetterlingen von paarungsbereiten weiblichen Tieren Sexuallockstoffe (= Pheromone) erzeugt und in die Umgebung ausgeschieden werden; männliche Schmetterlinge derselben Art können dann mit Hilfe dieses Riechstoffes die Weibchen finden.

Grundsätzlich gibt es drei verschiedene Möglichkeiten, Sexuallockstoffe im Pflanzenschutz anzuwenden:

Sogenannte Pheromonfallen, bestückt mit synthetischen Sexuallockstoffködern, werden in potentiellen Befallsgebieten ausgehängt. Der Fallenfang von männlichen Faltern erbringt den Nachweis des Auftretens des Schädlings. Außerdem lassen sich Hinweise zur Befallstärke und auf den richtigen Zeitpunkt der Bekämpfung ableiten.

Man kann den Lockstoff mit insektiziden Wirkstoffen kombinieren. Es besteht die Möglichkeit, dem Köder/der Falle, Insektizide zuzusetzen oder aber nur in unmittelbarer Umgebung der Falle zu behandeln. Damit dann der größte Teil der aus weiter Entfernung angelockten männlichen Falterbevölkerung abgetötet werden (Abfangtechnik). Die Biotopbelastung ist auf ein vertretbares Maß reduziert.

Schließlich kann der Schädling durch das Verfahren der Luftraumsättigung mit Sexuallockstoffen oder ähnlich wirkenden Substanzen bekämpft werden. Die männlichen Schmetterlinge werden am Auffinden der Weibchen gestört und somit die Paarung der Tiere verhindert. In diesem Fall wird im gesamten Bereich der zu schützenden Pflanzenkultur eine größere Menge des Lockstoffes gleichmäßig im Luftraum verteilt, so daß die Männchen überall die Gegenwart des Duftstoffes empfinden können und ihr normales Orientierungsverhalten gestört ist.

Selbst bei dieser letztgenannten Verfahrensweise der Anwendung von Sexuallockstoffen werden nur verhältnismäßig kleine Mengen der Wirkstoffe, welche oft nur Bruchteile der üblichen Dosen der klassischen Insektizidwirkstoffe entsprechen, benötigt. (Birch (ed.): Pheromones North Holland Publ. Co., 1974.)

Es handelt sich dabei um eine äußerst selektive, untoxische Bekämpfungsmethode unter größtmöglicher Schonung der Nicht-Zielorganismen, insbesondere der Nützlinge.

Es wurde nun gefunden, daß 5,9-Dimethylheptadecan einen wirksamen Lockstoff zur Anwendung auf Leucoptera scitella darstellt.

Gegenstand der Erfindung sind ferner Mittel zur Anlockung oder zur Verwirrung männlicher Tiere der Art Leucoptera scitella, die einen Gehalt an 5,9-Dimethylheptadecan aufweisen.

Ein möglicher Syntheseweg für 5,9-Dimethylheptadecan ist der im folgenden Herstellbeispiel beschriebene.

<u>Herstellbeispiel</u> (vgl. nachstehendes Schema)

Zu einer auf 0°C gekühlten etherischen Lösung von Laevulinsäureethylester wurde unter Stickstoff eine etherische Lösung der Grignardverbindung aus n-Chloroctan zugetropft. Das dadurch erhaltene Lacton, 5-Methyl-5-octyltetrahydrofuran-2-on (II) wird mit methanolischer Salzsäure in 4-Chlor-4-methyl-dodecansäuremethylester (III) umgewandelt, der nach thermischer Eliminierung von Salzsäure und anschließender Hydrierung racemischen 4-Methyldodecansäuremethylester (IV) liefert, aus dem durch

Verseifung 4-Methyldodecansäure (V) erhalten wird. Parallel wird aus 2-Hexanol das Tosylat hergestellt, das mit Natriummalonester in ethanolischer Lösung 2-Carbethoxy-3-methyl-heptansäurethylester (VI) bildet. Dieser wird nach Verseifung und thermischer Decarboxylierung in racemische 3-Methylheptansäure (VII) umgewandelt.

Das Pheromon, 5,9-Dimethylheptadecan, wird durch gemischte Kolbe-Elektrolyse aus 4-Methyldodecansäure (V) und 3-Methylheptansäure (VII) dargestellt: 3 g (V) und 2 g (VII) werden in 50 ml Methanol gelöst und mit Trimethylamin neutralisiert. Nun wird unter Verwendung von Pt-Elektroden mit einer Stromdichte von 0,3 Amp/$cm^2$ bei Raumtemperatur 2 h elektrolysiert. Das Methanol wird im Vakuum entfernt, der Rückstand wird in Ether aufgenommen und nacheinander mit H Cl, Na OH und $H_2O$ gewaschen und mit Mg $SO_4$ getrocknet. Nach Entfernen des Ethers wird das Produkt im Vakuum destilliert.

Kp 0,8 83°C Ausbeute 30 %

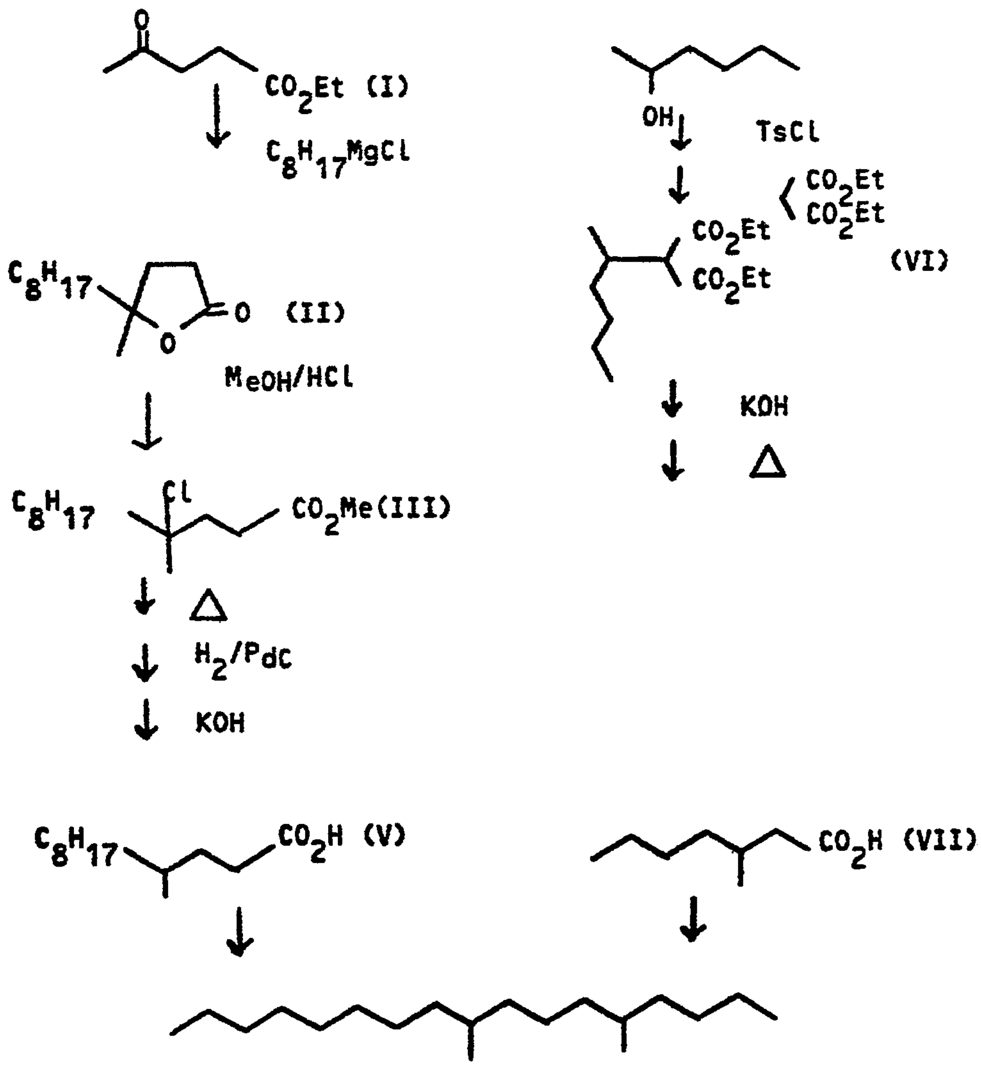


Zur erfindungsgemäßen Formulierung des Wirkstoffs kommen sowohl flüssige wie auch feste Präparationen in Frage.

Als Lösungsmittel kommen hochsiedende, aromatische, aliphatische oder cycloaliphatische Verbindungen in Betracht. Neben Kohlenwasserstoffen eignen sich Ester, Ether oder Ketone besonder gut. Typische Vertreter dieser Klassen sind z.B.: Xylol, Methylnaphthaline, Paraffinöle, Cyclohexanon, Ethylglykolacetat, Isophoron und Dibutylphthalat.

Zum Zwecke der Wirkungsverlängerung können Lösungen in pflanzlichen, tierischen oder synthetischen Ölen oder Fetten und anderen verdunstungshemmenden Lösungsmitteln mit niedrigem Dampfdruck (wie s.B. Dioctylphthalat) hergestellt werden.

Feste Zubereitungen erhält man dadurch, daß man den Wirkstoff in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe bindet; in Kunststoffkapseln oder -behältern sowie in mehrschichtigen Kunststoffplättchen, sogenannten Flakes, eingeschlossener Wirkstoff kann durch die Wände diffundieren; auch dadurch wird eine gleichmäßige Angabe an die Luft über längere Zeiträume hinweg erreicht. Außerdem kann der Wirkstoff aus geeigneten Behältern (Kapillaren oder anderen Gefäßen) durch enge Öffnungen zur Verdunstung gebracht werden, wodurch über längere Zeiträume hinweg besonders gleichmäßig Duftkonzentrationen erzielt werden.

Der Gehalt dieser Zubereitungen an Wirkstoff kann innerhalb weiter Grenzen schwanken. Generell kann das Verhältnis Wirkstoff : Zusatzstoff z.B. im Bereich von z.B. 10:1 bis 1:$10^3$ liegen. In Kapselformulierungen oder anderen geeigneten Behältern kann z.B. der Wirkstoff in reiner, unverdünnter Form angewendet werden und sein Gewichtsanteil, bezogen auf die Gesamtformulierung, sehr hoch sein und bis zu 90 % betragen. Im allgemeinen genügen jedoch sehr geringe Wirkstoffkonzentrationen in den Zubereitungen, um die gewünschte Wirkung auf Leucoptera-Männchen auszuüben. Bevorzugt ist ein Mengenverhältnis Wirkstoff: Zusatzstoff von 1:3 bis 1:$10^2$.

Man kann den Wirkstoff auch in vergleichsweise hohe Konzentrationen ausbringen, umd die Männchen durch Desorientierung und Konfusion nicht nur am Auffinden der Weibchen sondern auch unmittelbar an der Paarung zu hindern. Für diese Methode eignen sich am besten Formulierungen mit schwerflüchtigen Zusatzstoffen, die den Wirkstoff protrahiert abgeben, wie Gummi, Zellstoff, Wachse, Polymerisate oder verdunstungshemmende, schwerflüchtige Öle der Paraffine, sowie Formulierungen in Kapseln oder anderen Behältern (Kapillaren), die den Lockstoff entweder durch ihre Wandung oder durch enge Öffnungen abgeben. Die Wirkstoffkonzentration liegt hier im allgemeinen im Bereich von 10:1 bis 1:$10^3$.

Freilanduntersuchungen in einem potentiellen Befallsgebiet von Leucoptera scitella belegen die biologische Funktion der beanspruchten Pheromonkomponente.

Zu diesem Zweck wurden Gummi träger mit dem aufgeführten Duftstoff imprägniert und in Fallenkörpern vom Deltatyp mit Klebefläche plaziert. In festgelegten Zeitintervallen erfolgte die Auswertung durch Auszählen der in den Pheromonfallen gefangenen Leucoptera-Männchen. Die Experimente wurden 1985 in Ungarn in mehrfacher Wiederholung durchgeführt.

**Tabelle 1**

| Köderbeladung µg | gefangene männliche Falter von L. scitella | | | |
|---|---|---|---|---|
| | Falle 1 | 2 | 3 | Total |
| 1000 | 147 | 86 | 98 | 331 |
| 100 | 23 | 31 | 21 | 75 |
| 10 | 5 | 0 | 28 | 33 |
| 1 | 0 | 1 | 9 | 10 |

## Patentansprüche

1. 5,9-Dimethylheptadecan.
2. Mittel zur Bekämpfung der Fleckenminiermotte, Leucoptera scitella, enthaltend 5,9-Dimethylheptadecan als Lockstoff.
3. Mittel nach Anspruch 2, enthaltend übliche Zusatzstoffe und Hilfsmittel.
4. Verfahren zur Bekämpfung der Fleckenminiermotte, dadurch gekennzeichnet, daß man ein Mittel nach Anspruch 2 in Verbindung mit einem Insektizid anwendet.
5. Verfahren zur Bekämpfung der Fleckenminiermotte, dadurch gekennzeichnet, daß man mit einem Mittel nach Anspruch 2 das Auftreten der Fleckenminiermotte in an sich bekannter Weise feststellt und danach an sich bekannte Bekämpfungsmethoden anwendet.

## Claims

1. 5,9-Dimethylheptadecane.
2. A composition for controlling the pear leaf blister moth, Leucoptera scitella, containing 5,9-dimethylheptadecane as an attractant.
3. A composition as claimed in claim 2, containing conventional additives and assistants.
4. A method of controlling the pear leaf blister moth, wherein a composition as claimed in claim 2 is used in conjunction with an insecticide.
5. A method for controlling the pear leaf blister moth, wherein the presence of the pear leaf blister moth is established in a conventional manner using a composition as claimed in claim 2, and conventional control methods are then employed.

**Revendications**

1. 5,9-diméthylheptadécane.

2. Agent de lutte contre la mineuse tachetée, Leucoptera (= Cemiostoma) scitella, contenant du 5,9-diméthylheptadécane comme appât.

3. Agent selon la revendication 2 contenant des additifs et auxiliaires usuels.

4. Procédé de lutte contre la mineuse tachetée caractérisé par le fait qu'on utilise un agent selon la revendication 2 en combinaison avec un insecticide.

5. Procédé de lutte contre la mineuse tachetée caractérisé par le fait qu'avec un agent selon la revendication 2 on fixe de manière connue en soi la formation des mineuses tachetées et on applique ensuite des méthodes de lutte connues en soi.